# EUROPEAN PATENT APPLICATION

(11) **EP 1 346 726 A1**
(43) Date of publication of application: **24.09.2003**
(21) Application number: 01272319.3
(22) Date of filing: 25.12.2001
(51) Int. Cl.: A61K 35/78, A61K 31/015, A61K 31/01, A61K 31/216, A61K 31/522, A61K 7/46, A61K 7/00, A61K 7/50, A61P 25/02, A61P 25/28, A61P 3/04

(54) **SYMPATHETIC-ACTIVATING PERFUME COMPOSITION**

(30) Priority: 25.12.2000 JP 2000393457; 25.12.2000 JP 2000393470
(71) Applicant: SHISEIDO COMPANY, LTD., Chuo-ku, Tokyo 104-8010 (JP)
(72) Inventor: HAZE, Shinichiro, C/O SHISEIDO RESEARCH CENTER, Yokohama-Shi, Kanagawa 224-8558 (JP); SAKAI, Keiko, C/O SHISEIDO RESEARCH CENTER, Yokohama-Shi, Kanagawa 224-8558 (JP); GOZU, Yoko, C/O SHISEIDO RESEARCH CENTER, Yokohama-Shi, Kanagawa 224-8558 (JP); KOIZUMI, Yukiko, Yokohama-shi, Kanagawa 222-0032 (JP); HARIYA, Takeshi, C/O SHISEIDO RESEARCH CENTER, Yokohama-shi, Kanagawa 236-8643 (JP); SHIBATA, Michio, C/O SHISEIDO RESEARCH CENTER, Yokohama-shi, Kanagawa 236-8643 (JP); SAKIGUCHI, Takayuki, C/O SHISEIDO RESEARCH CENTER, Yokohama-shi, Kanagawa 224-8558 (JP)
(74) Representative: Santarelli
(86) International application number: JP0111398
(87) International publication number: WO02051428

(57) **Abstract**

Sympathetic nerve-stimulating fragrant compositions with weight-loss effects, characterized by containing one or more selected from among fennel oil, grapefruit oil, pepper oil, hyssop oil, sage oil, estragon oil, eucalyptus oil, rosemary oil, cinnamon oil, clove oil, ylang ylang oil, ginger oil, geranium oil and olibanum, or one or more from among limonene, pinene, myrcene and benzyl benzoate as the active ingredients in the oils, and preferably also containing caffeine.

## Description

### Technical Field

The present invention relates to fragrant compositions which stimulate sympathetic nerves. More specifically, it relates to sympathetic nerve-stimulating fragrances containing at least one type of essential oil.

### Background Art

The autonomic nervous system is divided into the sympathetic nervous system and the parasympathetic nervous system, based on functional differences, and it is involved in co-relative regulation of the functioning of various organs. Stimulation of sympathetic nerves is known to cause an increased cardiac rate and suppressed gastrointestinal activity, as well as other various effects, and such stimulation can be brought about by the action of hormones or the like and even by psychological stress. On the other hand, it is a traditionally well-known fact that fragrant substances can have various psychological effects, and calming fragrance, mood-enhancing fragrance and the like have long been known.

In recent years, the sedative and arousing effects of such fragrant substances have been confirmed by brain wave measurements and other means. The systemic influences, including psychological effects, of fragrant substances have come to be utilized in clinics throughout the world in the form of "aromatherapy", but among the fragrant substances with known effects on the autonomic nervous system, only a limited number of fragrant substances have been reported to stimulate the parasympathetic nervous system, and at the current time absolutely no fragrant substances have been found to act on the sympathetic nervous system (Japanese Unexamined Patent Publication HEI No. 11-209294).

### Disclosure of the Invention

Given the stressful nature of modern society, it has been a desirable to goal to provide sympathetic nervous system stimulants with minimal side-effects as autonomic nervous system regulators intimately involved in homeostatic control of the body.

As a result of diligent research directed toward solving the problem mentioned above, the present inventors have completed the present invention upon finding that the fragrance of certain types of fragrant substances have sympathetic nerve-stimulating effects.

Specifically, the present invention relates to sympathetic nerve-stimulating aromatics comprising at least one type of fragrant substance as an active ingredient, as well as to external skin applications and sundry goods characterized by containing the fragrant substances as active ingredients.

The fragrances or fragrant substances referred to here are preferably fennel oil, grapefruit oil, pepper oil, hyssop oil, sage oil, estragon oil, eucalyptus oil, rosemary oil, cinnamon oil, clove oil, ylang ylang oil, ginger oil, geranium oil and olibanum, while combinations of these fragrant substances may also be used. Limonene, pinene, myrcene or benzyl benzoate are also preferably included, and combinations of these fragrant substances may also be used.

The present invention therefore provides sympathetic nerve-stimulating fragrant compositions comprising one or more selected from among fennel oil, grapefruit oil, pepper oil, hyssop oil, sage oil, estragon oil, eucalyptus oil, rosemary oil, cinnamon oil, clove oil, ylang ylang oil, ginger oil, geranium oil and olibanum, or characterized by containing one or more from among limonene, pinene, myrcene and benzyl benzoate.

The invention further provides external skin applications and sundry goods comprising any of the aforementioned fragrant compositions as active ingredients.

The invention still further provides weight-loss fragrant compositions comprising one or more selected from among fennel oil, grapefruit oil, pepper oil, hyssop oil, sage oil, estragon oil, eucalyptus oil, rosemary oil, cinnamon oil, clove oil, ylang ylang oil, ginger oil, geranium oil and olibanum, or characterized by containing one or more from among limonene, pinene, myrcene and benzyl benzoate.

The invention still further provides memory-enhancing fragrant compositions comprising one or more selected from among fennel oil, grapefruit oil, pepper oil, hyssop oil, sage oil, estragon oil, eucalyptus oil, rosemary oil, cinnamon oil, clove oil, ylang ylang oil, ginger oil, geranium oil and olibanum, or characterized by containing one or more from among limonene, pinene, myrcene and benzyl benzoate.

The invention still further provides a weight-loss method characterized by exposure to the fragrance of one or more selected from among fennel oil, grapefruit oil, pepper oil, hyssop oil, sage oil, estragon oil, eucalyptus oil, rosemary oil, cinnamon oil, clove oil, ylang ylang oil, ginger oil, geranium oil and olibanum, or characterized by exposure to the scent of one or more from among limonene, pinene, myrcene and benzyl benzoate.

The invention yet further provides weight-loss compositions comprising the aforementioned sympathetic nerve-stimulating fragrant compositions as active ingredients. The compositions also preferably additionally contain caffeine.

### Brief Description of the Drawings

Fig. 1 is a graph showing sympathetic nerve relative activity by systolic blood pressure fluctuation analysis.
Fig. 2 is a graph showing sympathetic nerve relative activity by systolic blood pressure fluctuation analysis.
Fig. 3 is a graph showing sympathetic nerve relative activity of a pepper hot water extract.
Fig. 4 is a graph showing sympathetic nerve relative activity of a combination of essential oils with sympathetic nerve-stimulating effects (Perfume Formulation 1).
Fig. 5 is a graph showing the increase in blood catecholamines provoked by the fragrance of sympathetic nerve-stimulating aromatics.
Fig. 6 is a graph showing the synergistic effect of caffeine and noradrenaline on expression of the gene for uncoupling protein-3 in subcutaneous fat tissue.
Fig. 7 is a graph showing suppression of body weight increase by grapefruit oil.
Fig. 8 is a graph showing reduction of fatty tissue thickness and dermal tissue thickness by grapefruit oil.
Fig. 9 is a graph showing blood sugar reduction by grapefruit.

### Embodiments for Carrying Out the Invention

The present invention will now be explained in greater detail.

### Essential oil production methods

### Pepper oil

Pepper oil is obtained by drying the unripened berries of *Piper* plants belonging to the *Piperaceae* family and steam distilling them together with the blackened coats. It is widely used throughout the world as a food spice for the stimulating refreshing nature of its aroma (from "Kaori no Hyakka" [Encyclopedia of Aromas], edited by the Japan Aroma Society).

### Clove oil

Clove is an evergreen of the *Myrtaceae* family, known as "choji" in Chinese herbal medicine. The product obtained by picking the preflowering buds and then drying and steam distilling them is known as clove bud oil, while clove leaf oil is obtained by steam distillation of the leaves or twigs, and both are used as cosmetics and food fragrances. It is composed mainly of eugenol and has a woody, spicy aroma (from "Kaori no Hyakka" [Encyclopedia of Aromas], edited by the Japan Aroma Society).

### Estragon oil

Estragon is *Artemisia dracunculus* belonging to the family Asteraceae, and it is used in French escargot dishes. In Europe, North America and elsewhere the cultured terrestrial herb in the flowering stage is cut off and then dried and steam distilled to obtain the oil. The major component of the fragrance is estragole, which has a sweet anise-like fragrance (from "Kaori no Hyakka" [Encyclopedia of Aromas], edited by the Japan Aroma Society).

### Hyssop oil

Hyssop is a plant of the genus *Hyssopus,* family Labiatae, which has held traditional importance as a medicinal herb. The leaves and flowering spikes are cut off and steam distilled to obtain a refreshing oil ((from "Kaori no Hyakka" [Encyclopedia of Aromas], edited by the Japan Aroma Society).

### Sage oil

Sage is an herb classified as genus *Salvia,* family *Labiatae,* and is widely distributed throughout various regions of the world. The oil is obtained by steam distillation of the dried leaves, and it has a fresh herb-like fragrance. It is also widely used throughout the world as a food spice (from "Kaori no Hyakka" [Encyclopedia of Aromas], edited by the Japan Aroma Society).

### Eucalyptus oil

Eucalyptus, an evergreen belonging to the *Myrtaceae* family, is important as a lumber material. It is distributed throughout the tropical and subtropical zones, and the oil is obtained by steam distillation of the dried leaves and twigs (from "Kaori no Hyakka" [Encyclopedia of Aromas], edited by the Japan Aroma Society).

### Grapefruit oil

Grapefruit, known by the scientific name of *Citrus* paradis *Macf.,* is an evergreen of the Rutaceae family, produced in the islands of the West Indies. The fruit has a soothing sweet and bitter taste, and the fragrant substance is obtained during production of the juice. The major fragrant component is limonene which has a characteristic citrus fragrance (from "Kaori no Hyakka" [Encyclopedia of Aromas], edited by the Japan Aroma Society).

### Fennel oil

Fennel is a European perennial herb of the *Umbellifera* family, also known as anise, which has been used as a medicinal herb since prehistoric times. It was introduced into Japan during the Heian period, and is widely cultivated throughout the world. The fruit has fragrance and a sweet taste, while the essential oil is obtained by steam distillation. The major component of the essential oil is anethole, which has a characteristic sweet fragrance (from "Kaori no Hyakka" [Encyclopedia of Aromas], edited by the Japan Aroma Society).

### Rosemary oil

Rosemary oil is a fragrant component with a refreshing, sweet smell obtained by steam distillation of the leaves or flowers of an evergreen shrub of the *Labiatae* family (scientific name: *Rosmarinus* officinalis), which is widely distributed throughout countries of the Mediterranean coast. It is widely used as an active ingredient of medicines and as a flavoring or perfume fragrance (from "Kaori no Hyakka" [Encyclopedia of Aromas]).

### Ylang ylang oil

Ylang ylang oil is obtained by steam distillation of the flowers of a plant belonging to the Annonaceae family (*Canning odorata*), or by solvent extraction of the fresh leaves with petroleum ether or the like. It is one of the most important fragrant plants known from antiquity, and has a floral fragrance reminiscent of mild jasmine. It is used as a fragrance harmonizer for preparation of flower fragrances, and also as a food fragrance (from "Kaori no Hyakka" [Encyclopedia of Aromas]).

### Cinnamon oil

Cinnamon is an evergreen tree of the *Lauraceae* family grown in Southeast Asia, and its fragrance is characterized by a sweet fragrance and a somewhat astringent, irritating flavor. Cinnamon oil is obtained by crushing and steam distilling the bark, but the oil can also be obtained by steam distillation of the leaves or twigs. Different features can be brought out depending on the form obtained, and the oil products are used for a wide range of purposes including foods, cosmetics and medicines (from "Kaori no Hyakka" [Encyclopedia of Aromas]).

### Ginger oil

Ginger oil is obtained by drying and steam distilling the rhizome of the perennial plant ginger (*Zingiber officinale Roscoe*) of the *Zingiberaceae* family, which is also used directly for cooking. Subtle differences in flavor are found according to the region of production, and it is used in beverages, livestock meat products, seasonings and the like, depending on its properties (from "Kaori no Hyakka" [Encyclopedia of Aromas]).

### Geranium oil

Geranium is a plant belonging to the genus *Pelargonium*, family *Geraniaceae,* and geranium oil is obtained by collecting the branches and leaves before flowering has begun and steam distilling after about a day. It is among the 10 most important fragrant substances. The powerful rose-like smell has led to its use in all types of prepared fragrance, and since it is stable and undergoes no alteration of smell even in alkali solution, it is often used as a mixed fragrance in high-quality soaps and detergents.

### Olibanum

This is a resin exuded from the bark of arborets and small trees of *Boswellia carterii Birdw* and other plants belonging to the order *Geraniales,* family *Burseraceae,* which grow wild in the sterile soil of Northeast Africa, southern Arabia and India. It is also known as frankincense. The fragrance of the resin itself is weak, but when lit by a flame it emits a powerful characteristic fragrance which has been utilized from antiquity in religious ceremonies. The resinoid obtained by organic solvent extraction from the resin is used as a compounding fragrance for oriental preparations.

### Explanation of other raw materials

### Limonene

A representative monocyclic terpene, a colorless liquid, existing as dextrorotatory d(+) and levorotatory 1(-) optical isomers. The d-form is obtained by fractionation of the essential oil obtained by steam distillation of the rinds of oranges, lemons and the like, while the 1-form is obtained from mint oil (Gosei Koryo [Synthetic Aromas], Kagaku to Shohin Chishiki, by Indo, M., Kagaku Kogyo Nippo Publication).

### Pinene

A representative terpene-based hydrocarbon contained in numerous essential oils, and most abundant in coniferous plants.

### Myrcene

Produced in large amounts by thermal decomposition of β-pinene. It is found naturally in bay oil, verbena oil, hops oil, galbanum oil, lemongrass oil and the like. It is a colorless to light brown liquid with a sweet balsamic or resinoid aroma.

### Benzyl benzoate

Found in true balsam, Peru balsam, jasmine oil, cinnamon oil, cananga oil and the like. A colorless liquid with a slightly floral, sweet balsam-like fragrance. It is widely used as a retaining agent or modifier in heavy floral-based or oriental compounding fragrance, and as a solvent for balsam, resinoids and the like.

There are no particular restrictions on the amount of essential oil added according to the invention, and it may be appropriately selected depending on the type of essential oil chosen and its relationship with the other added components. It will normally be 0.001-100 wt% and preferably 10-100 wt% in a fragrant composition (perfume formulation).

There are no particular restrictions on the use of the composition of the invention, and it may be used as a fragrant composition for external applications, in cosmetics, medicines, quasi drugs and the like or in incenses, as a sundry fragrant composition, such as in a space dusting powder, or as a beverage or food aromatic composition.

In addition to the aforementioned sympathetic nerve-stimulating aromatics, other common fragrant components may be added depending on the specific form of the composition, so long as the effect of the invention is not impeded.

Sympathetic nerve stimulation is known to produce effects of weight loss and memory enhancement, and the aforementioned essential oil composition may also be used for these purposes.

The fragrant composition of the invention may be used alone, or it may be used in combination with other sympathetic nerve-stimulating agents, for example, capsaicin, zingerone and piperine, and/or other antiobestic or weight-loss components, for example, xanthine derivatives such as caffeine and the like, crude drugs such as Chinese tea, green tea extract, anise, peony, ginger, lavender, capsicum and the like, or tamarind, gymnema sylvestre, inosit, dextran sulfate and the like.

Particularly when the present invention is used for weight loss, the weight-loss effect can be further increased by its combination with caffeine. That is, the synergistic effect between caffeine and noradrenaline, which is secreted in the body upon stimulation of sympathetic nerves, causes burning of fat and therefore a higher weight-loss effect. This synergistic effect is clearly shown, for instance, in Examples 2 and 3. Example 2 demonstrates a clear increase in the level of catecholamines (adrenaline and noradrenaline) in the blood by the fragrant composition of the invention. Example 3 clearly demonstrates a synergistic increase in expression of uncoupling protein-3 by noradrenaline and caffeine.

As described in Japanese Patent Application Specification No.2001-258346, energy consumption (fat consumption) by uncoupling protein-3 leads to body weight reduction. It is reasonable to assume, therefore, that adding caffeine to the fragrant composition of the invention will produce a synergistic effect of weight loss.

A combination of the fragrant composition of the invention and caffeine may be a simple mixture thereof. When such a mixture is applied to the skin, the fragrant components volatilize and are detected through the nose while the caffeine is absorbed through the skin.

The amount of caffeine added as a drug agent is 0.001-5 wt%. The following may be used as a general standard for its proportions when used as a fragrant additive.

| Type of product | Aromatic additive proportion |
|---|---|
| Cosmetic water | 0.001-0.05 |
| Emulsion | 0.03-0.2 |
| Cream | 0.05-0.2 |
| Gel | 0.005-0.2 |
| Aerosol | 0.001-30 |
| Bath herb | 0.2-3 |
| Fragrance/Room fragrance | 1-100 |
| Incense | 1-30 |
| Foam pack | 0.03-0.2 |

Grapefruit oil increases sympathetic nerve stimulation, as indicated in Example 5 of the invention. As shown by Example 6, grapefruit oil has an antiobestic effect and is useful as an active ingredient for antiobestic agents and weight-loss agents. Example 7 demonstrates that grapefruit oil also has an effect of lowering blood sugar level, and is therefore useful as an active ingredient in hypoglycemic agents for aid in treatment of diabetes (routine care).

The amount of essential oil used according to the invention is not particularly restricted and may be appropriately selected depending on the type of essential oil chosen and its relationship with the other added components.

The composition of the invention may be used as an fragrant composition for external applications, in cosmetics, medicines, quasi drugs and the like or in incenses, as a sundry fragrant composition, such as in a space dusting powder, or as a beverage or food fragrant composition, and there are no particular restrictions on such purposes of use.

In addition to the aforementioned sympathetic nerve-stimulating aromatics, other common fragrant components may be added depending on the specific form of the composition, so long as the effect of the invention is not impeded.

The fragrant composition of the invention may be used alone, or it may be used in combination with other sympathetic nerve-stimulating agents, for example, capsaicin, zingerone and piperine, and/or other antiobestic or weight-loss components, for example, xanthine derivatives such as caffeine and the like, crude drugs such as Chinese tea, green tea extract, anise, peony, ginger, lavender, capsicum and the like, or tamarind, gymnema sylvestre, inosit, dextran sulfate and the like.

### Evaluation of autonomic nerve activity

Autonomic nerves are largely divided into sympathetic nerves and parasympathetic nerves, and they are responsible for internal organ and gland regulation. Adrenaline and acetylcholine released from nerve endings act directly on glands and muscles, antagonistically regulating the function of the heart, blood vessels, pupils, gastrointestine, sweat glands, etc. Consequently, numerous reports indicate that autonomic nerve activity can be measured by directly measuring adrenaline or acetylcholine levels, or by measuring cardiac rate, circulation and blood pressure, diaphoresis, pupil response and the like ("Seiri Shinrigaku" [Physiological Psychology], Fukumura Publications).

Any appropriate method of measurement may be used to measure the effects of aromas on the autonomic nervous system.

### Formulation Example

The oils of the invention or their components may be used alone without any additives, or they may be used in various forms such as in perfume products. As other components which are included in cosmetics there may be mentioned various powders such as talc, humectants, chelating agents, ultraviolet absorbers, dyes, preservatives, softening agents, solid or liquid anionic agents, cationic agents, non-ionic agents or ampholytic agents, high molecular agents, fats and oils, gums (for example, guar gum, xanthan gum or gum arabic), gelatin, microencapsulating carriers (for example, ureaformaldehyde copolymer), and the like.

The oil of the invention may be used alone or in combination with other components (alcohol solvents, aromatics or perfume bases), in a form absorbed in a porous polymer structure or in a form supported on cyclodextrin (α, β, γ) or hydroxyalkyl β-cyclodextrin. Room fragrance

| | |
|---|---|
| (1) Alcohol | 80% |
| (2) Purified water | remainder |
| (3) Antioxidant | 5% |
| (4) Mixed aroma | q.s. |
| (5) Grapefruit oil | 10% |

### Examples

The present invention will now be explained in greater detail by way of examples, with the understanding that the invention is in no way limited by the examples.

### Example 1. Measurement of sympathetic nerve activity

Cardiac rate intervals (heartbeat intervals) and blood pressure changes (fluctuations) are complex even during resting periods, and are governed by several systems including the sympathetic and parasympathetic nervous systems. It is known that frequency analysis of heartbeat intervals and blood pressure fluctuations can used to calculate the activity of each of these systems. The high-frequency component of cardiac rate fluctuation indicates parasympathetic nerve activity while the low-frequency component indicates the combined activity of the sympathetic and parasympathetic nervous systems, and the low-frequency component of blood pressure fluctuation indicates sympathetic nerve activity (from material by Dainippon Pharmaceutical Co.).

Sympathetic nerve activity was measured by analysis of systolic blood pressure fluctuation using an autonomic nervous system activity analysis system (FLUCLET, product of Dainippon Pharmaceutical Co.). Subjects were allowed to rest for 5 minutes in a thermostatic room at room temperature (24°C), the blood pressure value was measured on the upper arm and used to correct the blood pressure value at the wrist, and then the wrist blood pressure was continuously measured thereafter. Cotton was placed under the nostrils at the start of measurement, the blood pressure was measured for 3 minutes without fragrant substance, and then the nose cotton was impregnated with fragrant substance and the blood pressure was measured for 3 minutes. The low-frequency component of the systolic blood pressure fluctuation at the time of measurement was determined, the sympathetic nerve activity was calculated, and the relative sympathetic nerve activity with fragrance (later 3 minutes) compared to without fragrance (earlier 3 minutes) was determined to evaluate the effect of the fragrance on sympathetic nerves.

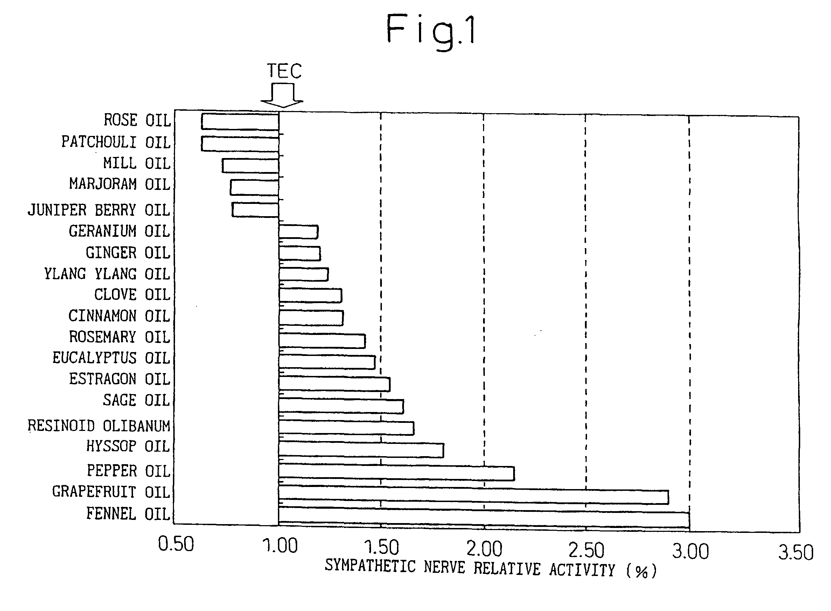

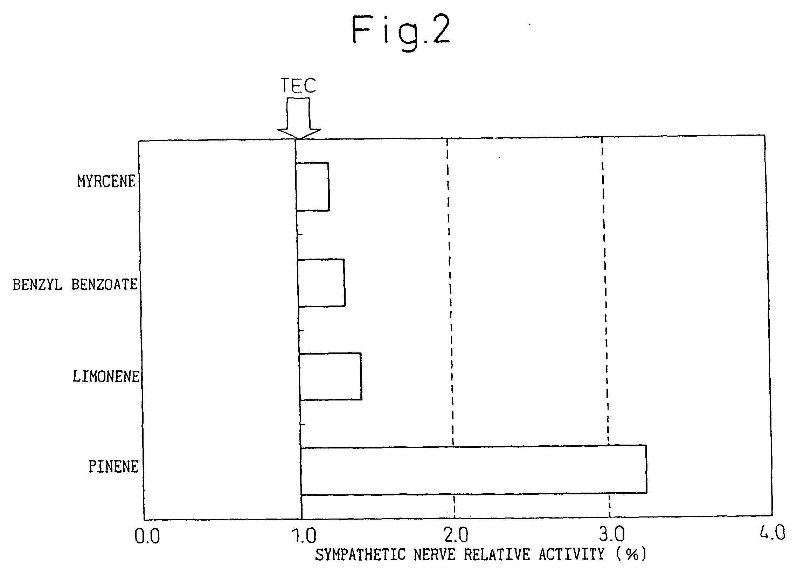

Fennel oil, grapefruit oil, pepper oil, hyssop oil, sage oil, estragon oil, eucalyptus oil, rosemary oil, cinnamon oil, clove oil, ylang ylang oil, ginger oil, geranium oil and olibanum exhibited sympathetic nerve stimulation of 1.2-3.0 times as compared with no fragrance (Fig. 1). TEC (triethyl citrate), which produces no detectable fragrance, showed no change in relative sympathetic nerve activity. As a result of GC/MS analysis of the fragrance which activated sympathetic nerves, limonene, pinene, myrcene and benzyl benzoate were found to be present as components in the effective fragrant substances. These fragrant components were also used to measure the sympathetic nerve activity by smelling of the fragrant substance by the same method, and an increased effect of sympathetic nerve activity was found (Fig. 2).

### Comparative Example 1

Rose oil, mill oil, marjoram oil, juniper berry oil and patchouli oil were found to have a sedative effect on sympathetic nerve activity (Fig. 1), in contrast to the essential oils with stimulating effects.

### Rose oil

The captivating and pleasant fragrance of rose is well known throughout the world. It has traditionally been known as the "queen of flowers". Rose oil is characterized by the potency and florid nature of its smell, as compared to rose absolute, and is often used as a floral base, spray, oriental base, etc. (from "Kaori no Hyakka" [Encyclopedia of Aromas]).

### Mill oil

A resin which exudes from the bark of plants primarily of the order *Geraniales,* family *Burseraceae,* genus *Commiphora.* Sixty species of *Commiphora* are known to exist in Africa and Arabia; when the sap seeping from a cut in the bark contacts with air it is converted to a deep, sweet, spicy, balsamic, lumpy resin. It is often used as a fragrant additive in oriental preparations, and is also used for flavoring in mouthwashes, toothpastes and the like to impart bitterness and pungency (from "Kaori no Hyakka" [Encyclopedia of Aromas]).

### Marjoram oil

Marjoram is a plant of the *Labiatae* family grown in the eastern Mediterranean coastal regions, and representative species are sweet marjoram (Origanum *marjorana*), Spanish wild marjoram, and the like. Marjoram is widely cultivated in the temperate zones and is used mainly as a spice. It is also used as a perfume, aromatic, cologne or oriental base.

### Juniper berry oil

Juniper is a species of evergreen tree of the *Cupressaceae* family (Juniperus *communis L*.) which reaches a height of 3 meters. It grows abundantly in the wild in Europe, Asia, North America and elsewhere. The oil is obtained from the two-year-old ripened dark blue fruit. The oil has a balsamic, woody, pine needle-like aroma, and is used to impart fragrance to wine and in drinks and breath fresheners (from "Kaori no Hyakka" [Encyclopedia of Aromas]).

### Patchouli oil

Patchouli is a perennial grass of the *Labiatae* family which grows in southern Asia, and whose fresh leaves are virtually scentless but produce a detectable smell when dried. The oil is obtained mainly by distillation in a direct flame system, and its powerful fragrance with a heavy oriental image is widely used for floral, citrus and other fragrant substances.

### Comparative Example 2

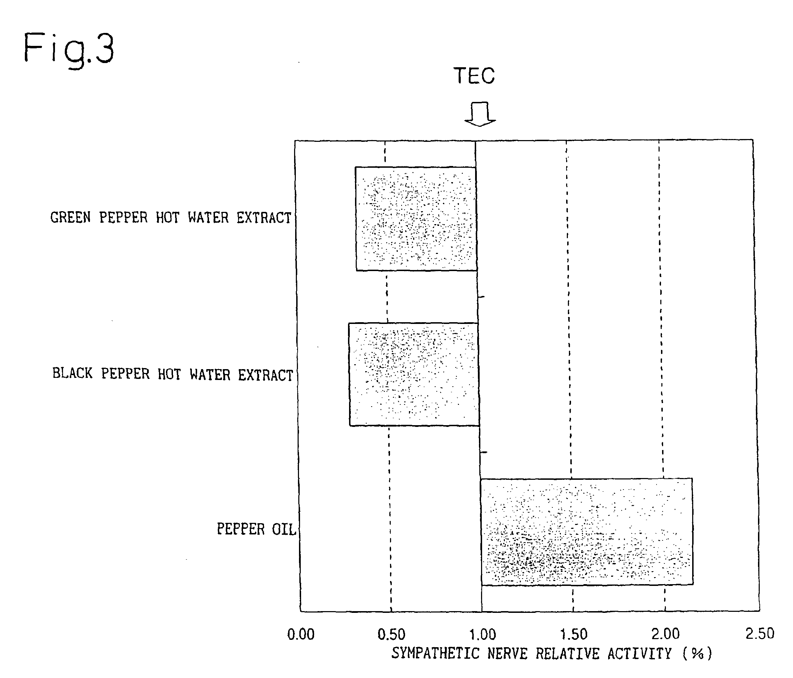

The effects of an essential oil fragrance, and fragrance of hot water extract on sympathetic nerve activity were compared. The essential oil used was pepper oil. The hot water extracts used were hot water extracts of black pepper and green pepper, prepared according to a common method. As a result, the fragrance of essential oil exhibited a notable effect, whereas the fragrance of hot water extract exhibited no effect, thus demonstrating that the fragrant components in the essential oil provided the main contribution to stimulation of the sympathetic nerves (Fig. 3).

### Example 2. Increase in blood catecholamines due to fragrance of sympathetic nerve-stimulating aromatics

Pepper oil and grapefruit oil were used as examples of fragrant substances which stimulate sympathetic nerves, in order to examine the changes in blood catecholamine levels produced by smelling of the fragrance of these fragrant substances. Twelve subjects were exposed to the smell of pepper oil or grapefruit oil, and the blood catecholamine levels were compared before and after 7 minutes of smelling of the fragrance. The subjects were seated and remained in a rested state for 30 minutes, after which 5 ml of blood was sampled as the pre-fragrance-exposure sample.

Essential oil-impregnated absorbent cotton was placed under the nostrils of the subjects while in a rested state to allow smelling of the fragrance, and after 7 minutes, 5 ml of blood was again sampled as the post-fragrance-exposure sample. The blood was sampled using an ADTA.2NA-containing sampling tube. Each blood sample was immediately centrifuged at 2000 rpm, 4°C for 10 minutes, and the plasma was separated and lyophilized until the time of measurement. The catecholamines in the plasma were measured by subjecting the deproteinated plasma sample to analysis with a catecholamine analyzer (HLC-8030, Toso Corp.) by post-column fluorescent reaction HPLC using diphenylethylene diamine, which reacts specifically with the catechol structure.

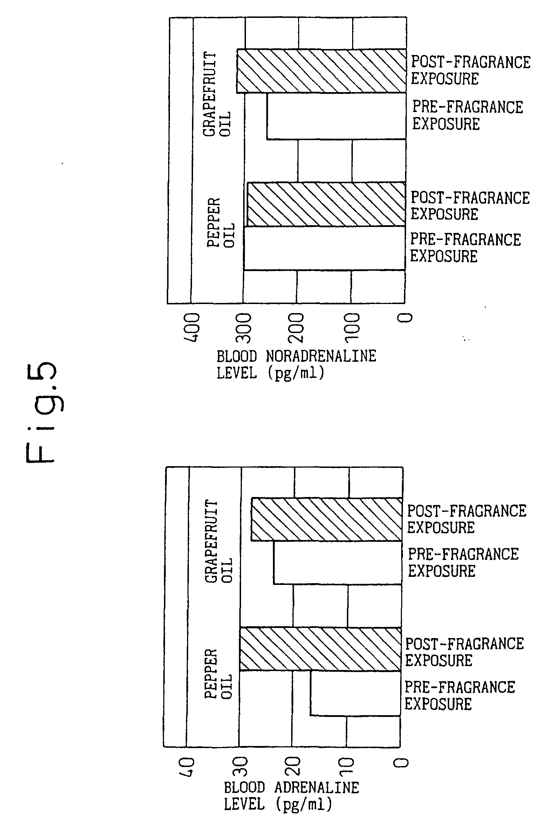

The results are shown in Fig. 5. As seen here, catecholamines such as adrenaline and noradrenaline increased after smelling the fragrance pepper or grapefruit fragrance, compared to pre-fragrance exposure, thus demonstrating that the smell of these fragrant substances stimulates sympathetic nerve activity and promotes secretion of catecholamines such as adrenaline and noradrenaline.

### Example 3. Synergistic effect of catecholamine (noradrenaline) and caffeine

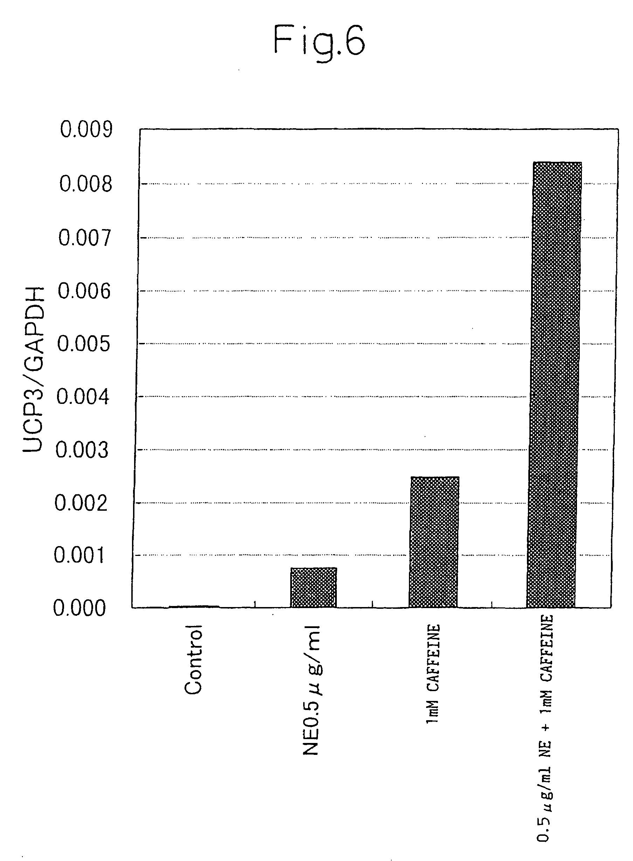

When noradrenaline (0.5 µg/ml) or caffeine (1 mM) was added to 70 mg of subcutaneous tissue from 6-week-old rats, the caffeine itself increased expression of uncoupling protein-3, as shown in Fig. 6, but when noradrenaline (0.5 µg/ml) and caffeine (1 mM) were added simultaneously, a synergistic increase in expression of uncoupling protein-3 was observed.

### Example 4. Verification of weight-loss effect by repeated use test

The emulsion described below (Formulation Example 5) was used to confirm a weight-loss effect. Twenty females aged 20-30 years old were asked to apply the emulsion to the body every morning and night for one month, and the changes in body weight and circumference (waist and hips) were compared. The results are shown in the following Table 1.

**Table 1**

| | Average before repeated use | Average after repeated use | Average reduction | Significant difference |
|---|---|---|---|---|
| Body weight | 60.45 Kg | 59.33 Kg | 1.12 Kg | *p<0.05 |
| Waist | 73.72 cm | 72.23 cm | 1.49 cm | *p<0.05 |
| Hips | 95.53 cm | 94.22 cm | 1.31 cm | *p<0.05 |

As clearly seen from Table 1, a significant reduction in body weight, waist circumference and hip circumference was found after repeated use, thus confirming the weight-loss effect of the invention.

### Example 5. Antiobestic effect of grapefruit oil

Normal mouse and obese mouse models were raised, and the changes in body weight and fatty tissue thickness after exposure to the fragrance of grapefruit oil were measured. The obese mouse models used were C57BL/KsJ-db/db male mice, which are commonly used as mouse diabetes models, and the controls used were C57BL/6 mice. For both types of mice, five were raised as 1 group, and a fragrance exposed group and control group were formed for each type, resulting 4 groups. The fragrance exposure method involved placing a dish in the cage, impregnating filter paper in 200 µL of grapefruit oil and covering it with wire mesh. The aroma exposure was carried out with 200 µl once each day, replacing the dish and filter paper on the next day.

### 1) Body weight

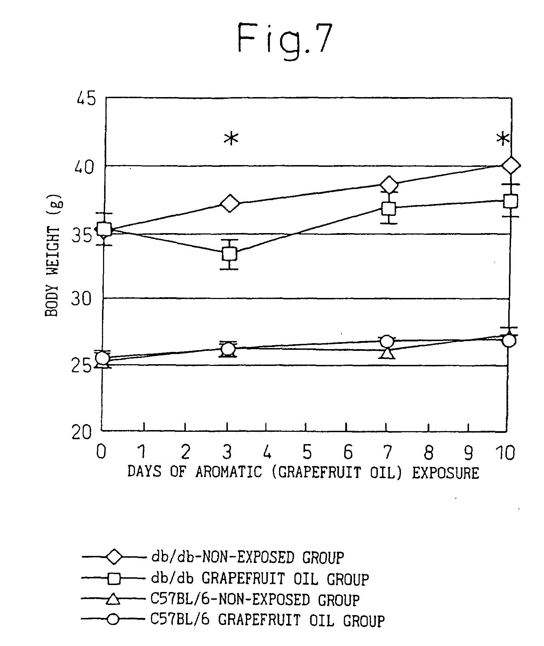

The body weight measurement was taken every 3 or 4 days, and observation was continued for 11 days after exposure to the fragrance, subsequent to a conditioning period. A significant decrease in body weight was found in the fragrance-exposed group within 3 days from the start of fragrance exposure. No difference was found in the body weight increase curves of the C57/BL6 control mice with or without fragrance. The body weight varied thereafter with a tendency to be lower in the fragrance-exposed group of obese mouse models, and a significant decrease in body weight was found even at measurement on the 11th day (Fig. 7).

### 2) Dermal and fatty tissue thickness

Subcutaneous tissue was sampled with a Dispopunch (6 mm: Maruho Co., Ltd.), and after fixing it with 10% neutral formalin, a tissue sample was prepared and stained with H&E dye (requested from Nack Co., Ltd.). The sample was observed with a microscope-connected image analyzer (OLYMPUS XL-10), and the dermal thickness and fatty tissue thickness were measured. Measurement was made at 5 locations of each sample, and the average was calculated as the value for that individual.

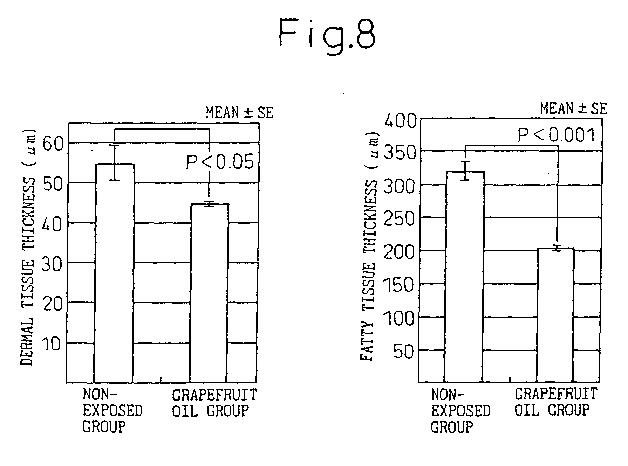

Fig. 8 shows the results of the dermal and fatty tissue thickness measurement using the analyzer. A notable reduction is clearly seen in the fatty acid tissue thickness, and a significant reduction in dermal thickness is also seen in the fragrance-exposed groups.

This confirmed that smelling of the fragrance of grapefruit suppressed body weight increase and reduced fatty tissue thickness in obese mice.

### Example 6. Effect of grapefruit oil on blood sugar

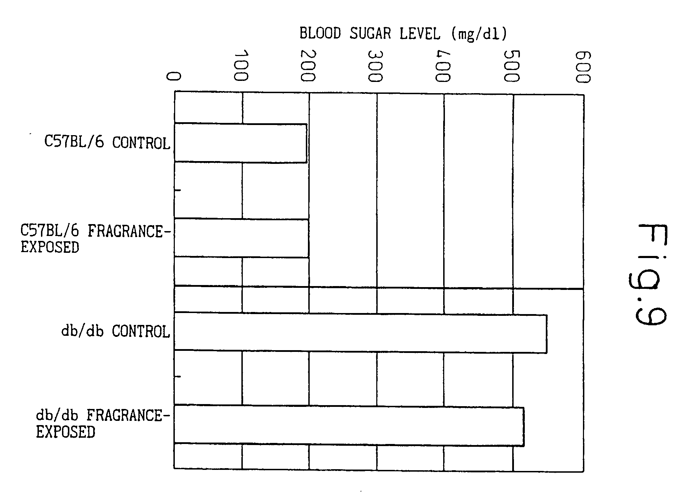

Normal mice (C57BL/6) and obese mice (C57BL/KSL-db/db) were separated into a grapefruit oil-exposed group and non-exposed group, with 5 mice in each group, and with exposure to grapefruit oil at 200 µL/group in the same manner as Example 2. The results, shown in Fig. 9, indicate that exposure to grapefruit oil lowered blood sugar levels in the obese mouse models.

### Preparation example

The "%" values in the preparation example indicate wt%.

| Fragrance | |
|---|---|
| (1) Alcohol | 75% |
| (2) Purified water | remainder |
| (3) Dipropylene glycol | 5% |
| (4) Aromatic of the invention | 10% |
| (5) Antioxidant | q.s. |
| (6) Grapefruit oil | 5% |
| (7) Dye | q.s. |
| (8) Ultraviolet absorber | q.s. |

Here, the "fragrance" is a solution of grapefruit oil of the invention in alcohol (for example, ethyl alcohol) or aqueous alcohol. The fragrance contains 1-99 wt% of the grapefruit oil of the invention. The proportion of water and alcohol is in a range from 50:50 to 0:100. The fragrance may also contain solubilizing agents, softening agents, humectants, thickeners, bacteriostatic agents or other materials commonly used for cosmetic products. Common perfuming means may be used for further addition and variation of fragrant components to harmonize with the grapefruit oil of the invention, r for greater retention or richness, or an appropriate combination of top note, middle note or last note fragrance may be included to prepare a mixture which periodically emits a captivating fragrance.

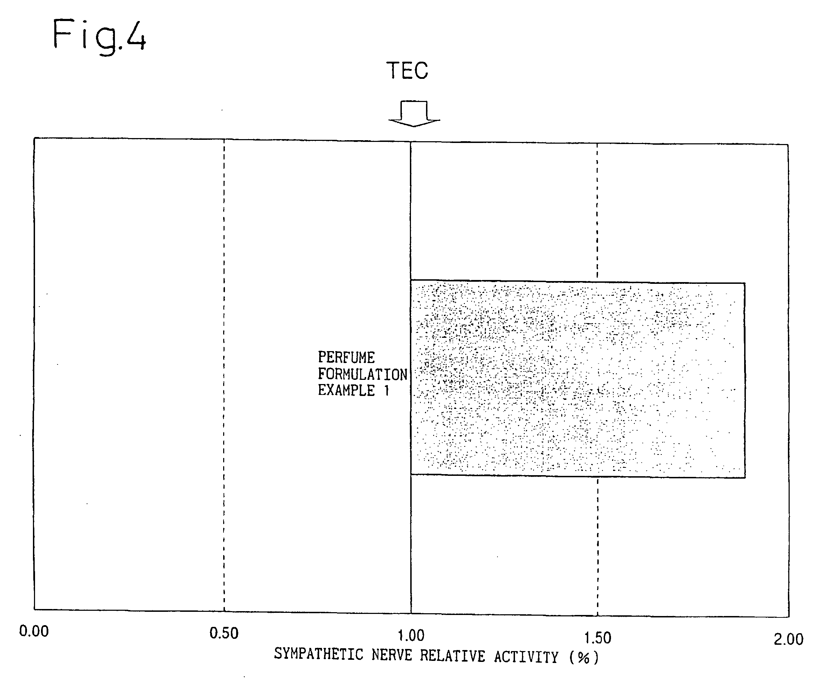

An example of a mixture combining essential oils which exhibit sympathetic nerve stimulation is the formulation listed as Perfume Formulation Example 1. The effect of this formulated fragrant substance on sympathetic nerve activity was compared and found to constitute a high sympathetic nerve-stimulating effect (Fig. 4).

| Perfume Formulation Example 1 | |
|---|---|
| (1) Grapefruit oil | 200 |
| (2) Linalool | 20 |
| (3) Linalyl acetate | 10 |
| (4) Pepper oil | 100 |
| (5) Fennel oil | 20 |
| (6) Estragon oil | 10 |
| (7) Sage oil | 20 |
| (8) Helional | 20 |
| (9) Manzanate | 20 |
| (10) Hedione | 150 |
| (11) Dihydromyrcenol | 50 |
| (12) Galaxolide | 50 |
| (13) Bergamot oil | 50 |
| (14) Limonene | 140 |
| (15) Cis-3-hexenol | 1 |
| (16) Helional | 2 |
| (17) Allylamyl glycolate | 2 |
| (18) Dipropylene glycol | 135 |
| Total | 1,000 |

| Perfume Formulation Examples 2 and 3 | | |
|---|---|---|
| No. Name of fragrances | Forml. Ex. 2 | Forml. Ex. 3 |
| (1) Grapefruit oil | 10.0 | 5.0 |
| (2) Estragon oil | 32.0 | 30.0 |
| (3) Fennel oil | 8.0 | 1.0 |
| (4) Pepper oil | 0.1 | - |
| (5) Hyssop oil | 0.1 | - |
| (6) Bergamot | 1.0 | - |
| (7) Cis-3-hexonol | 2.0 | 0.7 |
| (8) Cis-3-hexenyl acetate | 1.0 | 0.3 |
| (9) Triplal | 2.0 | 0.7 |
| (10) Tomato base | 3.0 | 1.0 |
| (11) Styrallyl acetate | 2.0 | 0.7 |
| (12) Rifarol | 3.0 | 1.0 |
| (13) Caron | 1.0 | 1.0 |
| (14) Linalool | 10.0 | 10.0 |
| (15) Benzyl acetate | 1.0 | 1.0 |
| (16) Hedione | 0.5 | 0.5 |
| (17) β-ionone | 1.0 | 1.0 |
| (18) Menthol | 10.0 | 10.0 |
| (19) Musk | 1.0 | 5.0 |
| (20) Triethyl citrate | 11.3 | 31.1 |

| Perfume Formulation Examples 4 and 5 | | |
|---|---|---|
| No. Name of fragrances | Forml. Ex. 4 | Forml. Ex. 5 |
| (1) Grapefruit oil | 200 | 200 |
| (2) Linalool | 20 | 20 |
| (3) Linalyl acetate | 10 | 20 |
| (4) Pepper oil | 100 | 20 |
| (5) Fennel oil | 20 | 10 |
| (6) Estragon oil | 10 | 10 |
| (7) Sage oil | 20 | 20 |
| (8) Helional | 20 | - |
| (9) Manzanate | 20 | - |
| (10) Hedione | 150 | 150 |
| (11) Dihydromyrcenol | 50 | - |
| (12) Galaxolide | 50 | 50 |
| (13) Bergamot oil | 50 | 50 |
| (14) Limonene | 140 | 140 |
| (15) Cis-3-hexenol | 1 | - |
| (16) Helional | 2 | - |
| (17) Allylamyl glycolate | 2 | - |
| (18) α-Hexylcinnamic aldehyde | - | 40 |
| (19) Phenylethyl alcohol | - | 20 |
| (20) Lilial | - | 20 |
| (21) Lyral | - | 20 |
| (22) β-ionone | - | 20 |
| (23) Methylionone-γ | - | 30 |
| (24) Geraniol | - | 30 |
| (25) Citronellol | - | 20 |
| (26) Dimethylbenzylcarbonyl | - | 50 |
| acetate | | |
| (27) Dipropylene glycol | 135 | 70 |
| Total | 1,000 | 1,000 |

### Skin application (cosmetic)

A skin application may be prepared using various drug agents suitable for use in skin applications, including extracts such as trimethylglycine, brown algae extract, red algae extract, green algae extract, tocopherol acetate, natto extract and houttuynia extract, as well as glycyrrhetic acid or the like.

| Cosmetic Water (Formulation Example 1) | |
|---|---|
| (1) Glycerin | 2% |
| (2) Dipropylene glycol | 2% |
| (3) PEG-60 hydrogenated castor oil | 0.3% |
| (4) Trimethylglycine | 0.1% |
| (5) Preservative | q.s. |
| (6) Chelating agent | q.s. |
| (7) Dye | q.s. |
| (8) Fragrant substance of the invention | q.s. |
| (9) Purified water | remainder |

| Cosmetic water (Formulation Example 2) | |
|---|---|
| (1) Alcohol | 30% |
| (2) Butylene glycol | 4% |
| (3) Glycerin | 2% |
| (4) PPG-13 Decyltetrades 24 | 0.3% |
| (5) Octylmethoxy cinnamate | 0.1% |
| (6) Menthol | 0.2% |
| (7) Tocopherol acetate | 0.1% |
| (8) Chelating agent | q.s. |
| (9) Dye | q.s. |
| (10) Fragrant substance of the invention | q.s. |
| (11) Purified water | remainder |

| Emulsion (Formulation Example 1) | |
|---|---|
| (1) Ethyl alcohol | 10% |
| (2) Cyclomethicone | 0.1% |
| (3) Butylene glycol | 5% |
| (4) Dimethicone | 3% |
| (5) Glycerin | 0.1% |
| (6' Menthol | 1% |
| (7) Trimethylsiloxysilicic acid | 0.1% |
| (8) Caffeine | 1% |
| (9) Trimethylglycine | 1% |
| (10) Xanthan gum | 0.001% |
| (11) Hydroxyethyl cellulose | 0.1% |
| (12) Soybean fermented extract | 1% |
| (13) Lauryl betaine | 0.5% |
| (14) Brown algae extract | 1% |
| (15) Red algae extract | 1% |
| (16) Green algae extract | 1% |
| (17) Carbomer | 0.2% |
| (18) Chelating agent | q.s. |
| (19) Paraben | q.s. |
| (20) Benzoic acid | q.s. |
| (21) Fragrant substance of the invention | q.s. |
| (22) Iron oxide | q.s. |
| (23) Caustic potash | 0.05% |
| (24) Dicalcium glycyrrhizinate | 0.01% |
| (25) Pyridoxine hydrochloride | 0.01% |
| (26) Glucoside ascorbate | 0.01% |
| (27) Ascorbic acid | 0.01% |
| (28) Arbutin | 3% |
| (29) Saxifraga extract | 0.1% |
| (30) Water | remainder |

| Emulsion (Formulation Example 2) | |
|---|---|
| (1) Butylene glycol | 4% |
| (2) Propylene glycol | 4% |
| (3) Carbomer | 0.2% |
| (4) Caustic potash | 0.2% |
| (5) Behenic acid | 0.5% |
| (6) Stearic acid | 0.5% |
| (7) Isostearic acid | 0.5% |
| (8) Glyceryl stearate | 1% |
| (9) Glyceryl isostearate | 1% |
| (10) Behenyl alcohol | 0.5% |
| (11) Batyl alcohol | 0.5% |
| (12) Squalane | 5% |
| (13) Trioctanoin | 3% |
| (14) Phenyltrimethicone | 2% |
| (15) Dicalcium glycyrrhizinate | 0.01% |
| (16) Preservative | q.s. |
| (17) Chelating agent | q.s. |
| (18) Pigment | q.s. |
| (19) Fragrant substance of the invention | q.s. |
| (20) Purified water | remainder |

| Emulsion (Formulation Example 3) | |
|---|---|
| (1) Glycerin | 3% |
| (2) Xylitol | 2% |
| (3) Carbomer | 0.1% |
| (4) Caustic potash | 0.1% |
| (5) Glyceryl isostearate | 1% |
| (6) Glyceryl stearate | 0.5% |
| (7) Behenyl alcohol | 1% |
| (8) Batyl alcohol | 1% |
| (9) Hardened palm oil | 2% |
| (10) Vaseline | 1% |
| (11) Squalane | 5% |
| (12) Erythrityl octanoate | 3% |
| (13) Cyclomethicone | 1% |
| (14) Preservative | q.s. |
| (15) Chelating agent | q.s. |
| (16) Fragrant substance of the invention | remainder |
| (17) Purified water | remainder |
| (18) Tranexamic acid | 1% |
| (19) Pantothenylethyl ether | 0.5% |
| (20) Nicotinamide | 0.1% |
| (21) Trehalose | 0.1% |
| (22) Rosemary extract | 0.1% |
| (23) Vitamin A | 0.1% |
| (24) Glycoside ascorbate | 0.001% |
| (25) Raspberry extract | 1% |
| (26) Scutellaria extract | 0.001% |
| (27) Cork tree extract | 0.01% |

| Emulsion (Formulation Example 4) | |
|---|---|
| (1) Ethanol | 2% |
| (2) Cyclomethicone | 10% |
| (3) Glycerin | 5% |
| (4) Dibutylene glycol | 1% |
| (5) Dimethicone | 1% |
| (6) Corn starch | 4% |
| (7) Mineral oil | 2% |
| (8) Trimethylsiloxysilicic acid | 5% |
| (9) Polyethylene glycol | 3% |
| (10) Menthyl lactate | 0.1% |
| (11) PEG-60 hydrogenated castor oil | 1% |
| (12) Aminopropyldimethicone | 1% |
| (13) Xanthan gum | 0.01% |
| (14) Tocopherol acetate | 0.01% |
| (15) Caffeine | 0.1% |
| (16) Sodium hyaluronate | 0.1% |
| (17) Xanthan gum | 0.01% |
| (18) Soybean fermented extract | 0.01% |
| (19) Hamamelis extract | 0.01% |
| (20) Houttuynia extract | 0.01% |
| (21) Carbomer | 0.3% |
| (22) Alkyl acrylate methacrylate copolymer | 0.2% |
| (23) HEDTA | q.s. |
| (24) Preservative | q.s. |
| (25) Fragrant substance of the invention | q.s. |
| (26) Pigment | q.s. |
| (27) Caustic potash | 0.15% |
| (28) Aminomethyl propanol | 0.05% |
| (29) Water | remainder |

| Emulsion (Formulation Example 5) | |
|---|---|
| (1) Ethanol | 15% |
| (2) Cyclomethicone | 6% |
| (3) Butylene glycol | 0.5% |
| (4) Dimethicone | 1% |
| (5) Glycerin | 1% |
| (6) Polyethylene glycol | 1% |
| (7) Menthyl lactate | 1% |
| (8) Menthol | 0.1% |
| (9) Trimethylsiloxysilicic acid | 1% |
| (10) Caffeine | 0.5% |
| (11) Trimethylglycine | 0.1% |
| (12) Xanthan gum | 0.1% |
| (13) Hydroxyethyl cellulose | 0.1% |
| (14) Soybean fermented extract | 0.01% |
| (15) Tocopherol acetate | 0.05% |
| (16) Lauryl betaine | 0.01% |
| (17) Brown algae extract | 0.01% |
| (18) Houttuynia extract | 0.01% |
| (19) Red algae extract | 0.01% |
| (20) Green algae extract | 0.01% |
| (21) Cellulose | 1% |
| (22) PEG-60 glyceryl isostearate | 1% |
| (23) Isostearic acid | 1% |
| (24) Carbomer | 0.1% |
| (25) Alkyl acrylate methacrylate copolymer | 0.1% |
| (26) EDTA | 0.1% |
| (27) Sodium metaphosphate | 0.1% |
| (28) Phenoxyethanol | 0.2% |
| (29) Paraben | 0.2% |
| (30) Fragrant substance of the invention | 0.45% |
| (Perfume Formulation Example 1) | |
| (31) Iron oxide (red) | 0.02% |
| (32) Menthyl glyceryl ether | 0.01% |
| (33) Water | remainder |

| Cream (Formulation Example 1) | |
|---|---|
| (1) Glycerin | 10% |
| (2) Butylene glycol | 5% |
| (3) Carbomer | 0.1% |
| (4) Caustic potash | 0.2% |
| (5) Stearic acid | 2% |
| (6) Glyceryl stearate | 2% |
| (7) Glyceryl isostearate | 2% |
| (8) Vaseline | 5% |
| (9) Stearyl alcohol | 2% |
| (10) Behenyl alcohol | 2% |
| (11) Hardened palm oil | 2% |
| (12) Squalane | 10% |
| (13) 4-methoxysalicylate K | 3% |
| (14) Preservative | q.s. |
| (15) Chelating agent | q.s. |
| (16) Pigment | q.s. |
| (17) Fragrant substance of the invention | q.s. |
| (18) Purified water | remainder |

| Cream (Formulation Example 2) | |
|---|---|
| (1) Glycerin | 3% |
| (2) Dipropylene glycol | 7% |
| (3) Polyethylene glycol | 3% |
| (4) Glyceryl stearate | 3% |
| (5) Glyceryl isostearate | 2% |
| (6) Stearyl alcohol | 2% |
| (7) Behenyl alcohol | 2% |
| (8) Liquid paraffin | 7% |
| (9) Cyclomethicone | 3% |
| (10) Dimethicone | 1% |
| (11) Octyl methoxycinnamate | 0.1% |
| (12) Hyaluronate Na | 0.05% |
| (13) Preservative | q.s. |
| (14) Chelating agent | q.s. |
| (15) Pigment | q.s. |
| (16) Fragrant substance of the invention | q.s. |
| (17) Purified water | remainder |

| Gel | |
|---|---|
| (1) Ethyl alcohol | 10% |
| (2) Glycerin | 5% |
| (3) Butylene glycol | 5% |
| (4) Carbomer | 0.5% |
| (5) Aminomethyl propanol | 0.3% |
| (6) PEG-60 hydrogenated castor oil | 0.3% |
| (7) Menthol | 0.02% |
| (8) Preservative | q.s. |
| (9) Chelating agent | q.s. |
| (10) Fragrant substance of the invention | q.s. |
| (11) Purified water | remainder |

| Aerosol (Formulation Example 1) | |
|---|---|
| (1) Glycerin | 2% |
| (2) Dipropylene glycol | 2% |
| (3) PEG-60 hydrogenated castor oil | 0.3% |
| (4) HPPCD | 1.0% |
| (hydroxypropyl β-cyclodextrin) | |
| (5) Preservative | q.s. |
| (6) Chelating agent | q.s. |
| (7) Dye | q.s. |
| (8) Fragrant substance of the invention | q.s. |
| (9) Purified water | remainder |
| (10) Nitrogen gas | 0.8% |

| Aerosol (Formulation Example 2) | |
|---|---|
| (1) Alcohol | 15% |
| (2) Butylene glycol | 2% |
| (3) Glycerin | 1% |
| (4) PPG-13 Decyltetrades 24 | 0.1% |
| (5) Silver-supported zeolite | 1.0% |
| (6) Chelating agent | q.s. |
| (7) Dye | q.s. |
| (8) Fragrant substance of the invention | q.s. |
| (9) Purified water | remainder |
| (10) LPG | 40% |

| Aerosol (Formulation Example 3) | |
|---|---|
| (1) Ethanol | 60% |
| (2) Menthyl lactate | 0.1% |
| (3) Sodium lactate | 0.1% |
| (4) Tocopherol acetate | 0.01% |
| (5) Lactic acid | 0.01% |
| (6) Caffeine | 0.01% |
| (7) Anise extract | 1% |
| (8) Hamamelis extract | 1% |
| (9) Houttuynia extract | 1% |
| (10) Dipropylene glycol | 1% |
| (11) Nitrogen gas | 0.9% |
| (12) Polyoxyethylene polyoxypropylene decyltetradecyl ether | 1% |
| (13) Butylene glycol | 2% |
| (14) Tocopherol | 0.05% |
| (15) Fragrant substance of the invention | q.s. |
| (16) PEG-60 hydrogenated castor oil | 0.1% |
| (17) Water | remainder |

| Bath herb | |
|---|---|
| (1) Sodium sulfate | 45.0% |
| (2) Sodium bicarbonate | 45.0% |
| (3) Hyssop oil | 10.0% |
| (4) Fragrant substance of the invention | q.s. |

| Room fragrance | |
|---|---|
| (1) Alcohol | 80.0% |
| (2) Purified water | remainder |
| (3) Antioxidant | 5.0% |
| (4) Fragrant substance of the invention | q.s. |
| (5) 3-methyl-3-methoxybuthanol | 5.0% |
| (6) Dibenzylidene sorbitol | 5.0% |
| (7) Sage oil | 5.0% |

| Incense | |
|---|---|
| (1) Tabu powder | 75.5% |
| (2) Sodium benzoate | 15.5% |
| (3) Fragrant substance of the invention | q.s. |
| (4) Eucalyptus oil | 1.0% |
| (5) Fennel oil | 1.0% |
| (6) Purified water | remainder |

| Massage gel | |
|---|---|
| (1) Erythritol | 2.0% |
| (2) Caffeine | 5.0% |
| (3) Cork tree extract | 3.0% |
| (4) Glycerin | 50.0% |
| (5) Carboxyvinyl polymer | 0.40% |
| (6) Polyethylene glycol 400 | 30.0% |
| (7) Trisodium ethylenediaminehydroxyethyl triacetate | 0.1% |
| (8) Polyoxyethylene (10) methylpolysiloxane copolymer | 2.0% |
| (9) Squalane | 1.0% |
| (10) Fennel oil | 1.0% |
| (11) Potassium hydroxide | 0.15% |
| (12) Purified water | remainder |

| Foam pack | |
|---|---|
| (1) Caffeine | 1.0% |
| (2) Sodium metaphosphate | 0.02% |
| (3) Trehalose | 2.0% |
| (4) Glycerin | 7.0% |
| (5) Methylparaben | 0.11% |
| (6) Potassium hydroxide | 0.15% |
| (7) Stearic acid | 0.5% |
| (8) Myristic acid | 1.0% |
| (9) Batyl alcohol | 1.5% |
| (10) Polyoxyethylene (60) hardened castor oil | 3.0% |
| (11) pepper oil | 0.3% |
| (12) Liquefied petroleum gas | 6.0% |
| (13) Dimethyl ether | 3.0% |
| (14) Fragrant substance of the invention | q.s. |
| (15) Purified water | remainder |

| Incense: | |
|---|---|
| (1) Tabu powder | 75% |
| (2) Sodium benzoate | 15% |
| (3) Perfume | q.s. |
| (4) Grapefruit oil | 5% |
| (5) Purified water | remainder |

| Toner | |
|---|---|
| (1) Glycerin | 4.0% |
| (2) 1,3-butylene glycol | 4.0% |
| (3) Tocopherol acetate | 0.05% |
| (4) Ethanol | 7.0% |
| (5) Polyoxyethylene (18) oleyl alcohol ether | 0.5% |
| (6) Methylparaben | 0.2% |
| (7) Citric acid | 0.05% |
| (8) Sodium citrate | 0.1% |
| (9) Trisodium ethylenediaminetetraacetate | 0.02% |
| (10) Grapefruit oil | 0.03% |
| (11) Purified water | remainder |

| Emulsion | |
|---|---|
| (1) Stearic acid | 1.5% |
| (2) Cetyl alcohol | 0.5% |
| (3) Beeswax | 2.0% |
| (4) Polyoxyethylene (10) monooleate | 1.0% |
| (5) Octyl methoxycinnamate | 2.0% |
| (6) Caffeine | 0.2% |
| (7) Sodium hyaluronate | 0.01% |
| (8) Triethanolamine | 0.75% |
| (9) Glycerin | 7.0% |
| (10) Inosit | 0.5% |
| (11) 1,3-butyleneglycol | 0.5% |
| (12) Trisodium ethylenediaminehydroxyethyl triacetate | 0.01% |
| (13) Ethylparaben | 0.3% |
| (14) Grapefruit oil | 0.05% |
| (15) Purified water | remainder |

| Massage cream | |
|---|---|
| (1) Solid paraffin | 5.0% |
| (2) Beeswax | 10.0% |
| (3) Vaseline | 15.0% |
| (4) Liquid paraffin | 41.0% |
| (5) 1,3-butylene glycol | 4.0% |
| (6) Glycerin monostearate | 2.0% |
| (7) POE (20) sorbitan monolaurate | 2.0% |
| (8) Borax | 0.2% |
| (9) Caffeine | 2.0% |
| (10) Preservative | q.s. |
| (11) Antioxidant | q.s. |
| (12) Grapefruit oil | 1.0% |
| (13) Purified water | remainder |

Components (5), (8) and (9) were added to component (13) and the mixture was heated to 70°C. After heating the oil portion to dissolution, components (6), (7), (10) and (11) were added and the mixture was adjusted to 70°C. This was gradually added to the aqueous phase prepared previously and subjected to pre-emulsification. After homogenizing the emulsified particles with a homomixer, they were deaired, filtered and cooled.

| Pack mask: | |
|---|---|
| (1) Polyvinyl alcohol | 15.0% |
| (2) Carboxymethyl cellulose | 5.0% |
| (3) 1,3-butylene glycol | 5.0% |
| (4) Ethanol | 12.0% |
| (5) Grapefruit oil | 0.1% |
| (6) Preservative | q.s. |
| (7) Buffer | q.s. |
| (8) POE oleyl alcohol ether | 0.5% |
| (9) Purified water | remainder |

Components (7) and (3) were added to component (9) and the mixture was heated to 70-80°C. Components (2) and (1) were then added thereto and stirred to dissolution. After adding and dissolving components (5), (6) and (8) in ethanol, the solution was added to the aforementioned aqueous phase for solubilization. The mixture was then deaired, filtered and cooled.

### Industrial Applicability

According to the present invention it is possible to provide sympathetic nerve-stimulating compositions with no side-effects, as well as weight-loss compositions and memory enhancing compositions.

## Claims

1. A sympathetic nerve-stimulating fragrant composition comprising one or more selected from among fennel oil, grapefruit oil, pepper oil, hyssop oil, sage oil, estragon oil, eucalyptus oil, rosemary oil, cinnamon oil, clove oil, ylang ylang oil, ginger oil, geranium oil and olibanum.

2. A sympathetic nerve-stimulating fragrant composition comprising one or more from among limonene, pinene, myrcene and benzyl benzoate.

3. An external skin application or sundry goods **characterized by** containing a sympathetic nerve-stimulating fragrant composition according to claim 1 or 2 as an active ingredient.

4. A weight-loss fragrant composition comprising one or more selected from among fennel oil, grapefruit oil, pepper oil, hyssop oil, sage oil, estragon oil, eucalyptus oil, rosemary oil, cinnamon oil, clove oil, ylang ylang oil, ginger oil, geranium oil and olibanum.

5. A weight-loss aromatic composition comprising one or more from among limonene, pinene, myrcene and benzyl benzoate.

6. A memory-enhancing fragrant composition comprising one or more selected from among fennel oil, grapefruit oil, pepper oil, hyssop oil, sage oil, estragon oil, eucalyptus oil, rosemary oil, cinnamon oil, clove oil, ylang ylang oil, ginger oil, geranium oil and olibanum.

7. A memory-enhancing fragrant composition comprising one or more from among limonene, pinene, myrcene and benzyl benzoate.

8. A weight-loss method **characterized by** exposure to the fragrance of one or more selected from among fennel oil, grapefruit oil, pepper oil, hyssop oil, sage oil, estragon oil, eucalyptus oil, rosemary oil, cinnamon oil, clove oil, ylang ylang oil, ginger oil, geranium oil and olibanum.

9. A weight-loss method **characterized by** exposure to the fragrance of one or more selected from among limonene, pinene, myrcene and benzyl benzoate.

10. A weight-loss composition comprising a sympathetic nerve-stimulating fragrant composition according to claim 1 or 2 as an active ingredient.

11. A weight-loss composition according to claim 10, **characterized by** further containing caffeine.
